# EUROPEAN PATENT APPLICATION

(11) **EP 4 194 014 A1**
(43) Date of publication of application: **14.06.2023**
(21) Application number: 21853594.6
(22) Date of filing: 29.07.2021
(51) Int. Cl.: A61L 2/10, A61L 2/24, H05B 47/115, H05B 47/13, H05B 47/16, H05B 47/165, H05B 47/17, A61L 9/20

(54) **DEVICE FOR INACTIVATING BACTERIA OR VIRUSES AND TREATMENT METHOD FOR INACTIVATING BACTERIA OR VIRUSES**

(30) Priority: 05.08.2020 JP 2020133323; 25.08.2020 JP 2020141843; 25.11.2020 JP 2020195381; 25.11.2020 JP 2020195386; 30.04.2021 JP 2021077287
(71) Applicant: Ushio Denki Kabushiki Kaisha, Tokyo 100-8150 (JP)
(72) Inventor: OKUMURA,Yoshihiko, Tokyo 100-8150 (JP)
(74) Representative: Tomerius, Isabel
(86) International application number: PCT/JP2021/028022
(87) International publication number: WO 2022/030342

(57) **Abstract**

Provided is an inactivation device for bacteria and/or viruses and a method of inactivation treatment for bacteria and/or viruses. The device and the method are capable of efficiently inactivating bacteria and/or viruses present in a space. The inactivation device for bacteria and/or viruses includes a light source section configured to emit ultraviolet light having a peak wavelength in a range from 190 nm or more to less than 240 nm, and a controller configured to control the light source section to execute a main operation mode including a first lighting operation and a second lighting operation in which ultraviolet light is emitted with lower light intensity than that of the first lighting operation.

## Description

### TECHNICAL FIELD

The present invention relates to an inactivation device for bacteria and/or viruses, particularly an inactivation device for bacteria and/or viruses using ultraviolet light. The present invention also relates to a method of inactivation treatment for bacteria and/or viruses.

### BACKGROUND ART

Conventionally, technologies for inactivating bacteria and/or viruses by irradiating them with ultraviolet light are known. In most cases, ultraviolet light with a wavelength of around 254 nm using a low-pressure mercury vapor lamp or the like as a light source, is utilized because DNA exhibits the highest absorption property at a wavelength of around 260 nm. The method of inactivation treatment for bacteria and/or viruses with ultraviolet light is characterized by the fact that sterilization can be performed simply by irradiating a space to be treated or objects to be treated with ultraviolet light, without spraying chemicals or other agents.

However, ultraviolet light in certain wavelength bands is known to pose a risk of adversely affecting human bodies when it radiates to human bodies. Hence, methods and devices of inactivating bacteria and/or viruses present in the space are being considered such that ultraviolet light avoids radiating to persons.

Patent Document 1, for example, discloses a germicidal lamp that is mounted on the ceiling in the space and, in the presence of persons, emits ultraviolet light toward the ceiling in a manner that ultraviolet light emitted from the germicidal lamp does not directly radiate to persons in the space.

### CITATION LIST

### PATENT DOCUMENTS

Patent Document 1: JP-A-2019-150668

### SUMMARY OF INVENTION

### Technical Problem

However, the sterilization lamps described in the above patent document 1 can only irradiate the area near the ceiling with ultraviolet light when persons frequently enter and leave the space or when persons work in the space for a long time, performing the inactivation treatment in a very narrow area and an area persons fail to reach.

In addition, the inactivation treatment in which ultraviolet light radiates away from persons cannot inactivate bacteria and/or viruses that adhere to and move around persons. Hence, such inactivation treatment is inefficient because bacteria and/or viruses that are carried through persons may adhere to the treated area immediately after the treatment has been completed.

In the viewpoints of the above problems, it is an object of the present invention to provide an inactivation device for bacteria and/or viruses and a method of inactivation treatment for bacteria and/or viruses, while ensuring the safety of persons and animals, the device and the method being capable of efficiently inactivating bacteria and/or viruses present in a space.

### Solution to the problem

An inactivation device for bacteria and/or viruses is an inactivation device for bacteria and/or viruses present in a space and includes:
a light source section configured to emit ultraviolet light having a peak wavelength in a range from 190 nm or more to less than 240 nm, and
a controller configured to control the light source section to execute a main operation mode including a first lighting operation and a second lighting operation in which ultraviolet light is emitted with lower light intensity than that of the first lighting operation.

In the present specification, "inactivation" refers to a concept that encompasses eliminating bacteria and/or viruses or causing them to lose their infectivity or toxicity, and "bacteria" refers to microorganisms such as bacteria and fungi (mold). Hereinafter, "bacteria and/or viruses" may be collectively referred to as "pathogens".

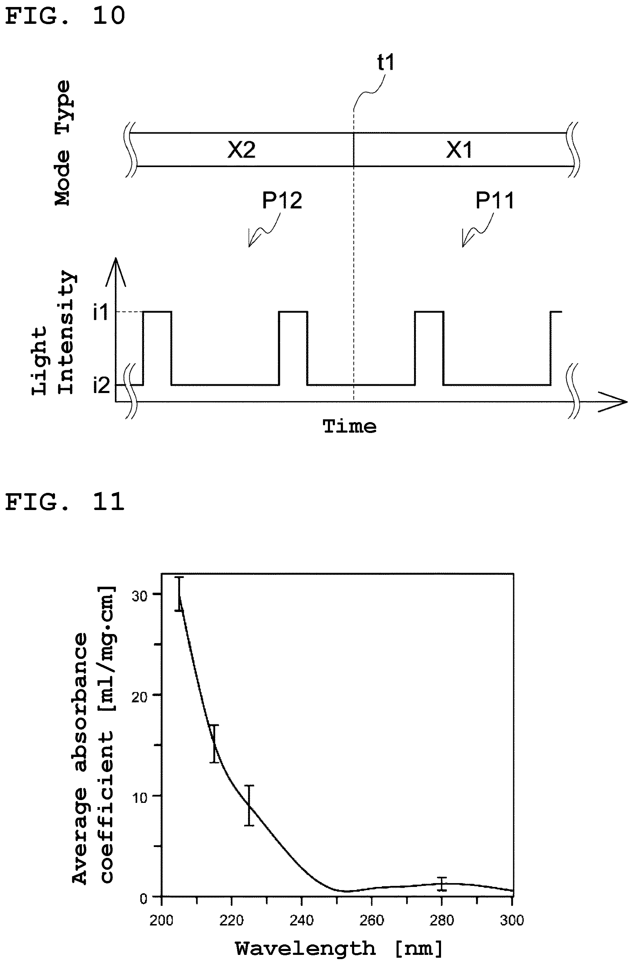

FIG. 11 is a graph illustrating the absorbance characteristics of proteins in the ultraviolet light area. FIG. 11 shows that proteins are less likely to absorb ultraviolet light having a wavelength of 240 nm or more, and more likely to absorb ultraviolet light having a wavelength of 240nm or less toward 200nm. Ultraviolet light having a wavelength of 240 nm or more readily transmits human skin and penetrates the skin. Hence, cells inside human skin are susceptible to damage. In contrast, ultraviolet light having a wavelength of less than 240 nm is easily absorbed by the front surface of human skin (e.g., stratum corneum) and is unlikely to penetrate the skin. Therefore, it is safer for the skin.

Moreover, as for eyes, ultraviolet light having a wavelength of less than 240 nm is unlikely to transmit corneas, thus it can be said that ultraviolet light is safer as its wavelength is shorter.

On the other hand, the presence of ultraviolet light having a wavelength of less than 190 nm allows oxygen molecules present in the atmosphere to undergo photolysis to produce a large number of oxygen atoms, leading to generating a large amount of ozone due to the bonding reaction between oxygen molecules and oxygen atoms. Hence it is undesirable that ultraviolet light having a wavelength of less than 190 nm is made to be emitted in the atmosphere.

Therefore, it can be said that ultraviolet light having a wavelength from 190 nm or more to less than 240 nm is ultraviolet light that is safe for humans and animals. From the viewpoint of enhancing safety for humans and animals, ultraviolet light emitted from the light source section preferably has a wavelength in a range from 190 nm or more to 237 nm or less, more preferably has a wavelength in a range from 190 nm or more to 235 nm or less, and especially more preferably has a wavelength in a range from 190 nm or more to 230 nm or less.

Applicable products according to the present invention can provide the capabilities of inactivating bacteria and/or viruses, which are inherent to ultraviolet light, without causing erythema or keratitis on the skin or eyes of humans or animals. In particular, unlike conventional light sources that emit ultraviolet light, the applicable products are characterized by their use in a manned environment, thus they can be mounted in an indoor or outdoor maned environment to emit the entire environment, providing virus control and sterilization in the air and on the surfaces of objects mounted in the environment.

This addresses Goal 3 of the UN-led Sustainable Development Goals (SDGs): "Ensuring healthy lives and promote welfare for all people of all ages" and contributes significantly to Target 3.3 "By 2030, end the epidemics of AIDS, tuberculosis, malaria and neglected tropical diseases and combat hepatitis, water-borne diseases and other communicable diseases".

As of the filing date of the present application, the ACGIH (American Conference of Governmental Industrial Hygienists) standard and JIS Z 8812 (Measuring Methods of Eye-hazardous Ultraviolet Radiation), for example, sets the threshold limit value (TLV) for the irradiation amount of ultraviolet light per 8 hours to human bodies in accordance with the wavelength band. In other words, upon the use of ultraviolet light in an environment where humans are present, it is recommended that the radiation intensity and lighting time of the light source section are determined such that the cumulative irradiation amount of ultraviolet light emitted in a given time is within the TLV standard values.

These regulations also specify the allowable limits for ultraviolet light having a wavelength from 190 nm or more to less than 240 nm. Hence, although the inactivation treatment is performed using ultraviolet light having a wavelength from 190 nm or more to less than 240 nm, which poses significantly low risk, it is desirable that the ultraviolet light radiates to persons in a manner that does not exceed the threshold limit values.

The present inventor, through diligent study, has found that it is effective to temporarily reduce the intensity of the ultraviolet light emitted from the light source section in order to reduce the irradiation amount of ultraviolet light to which a person is exposed.

The above configuration enables the inactivation device for bacteria and/or viruses with little risk of adversely affecting human bodies. In addition, the inactivation device in the above configuration can continue the inactivation treatment in the space where persons come and go while controlling the irradiation amount of ultraviolet light to the persons, even in the case in which persons frequently enter and leave the space, or the case in which persons work in the space for a long period of time.

Also, in the case in which no person is present in the space 2, this configuration eliminates the need for unnecessarily continuing ultraviolet light irradiation with high intensity, leading to, for example, reducing the power consumption, and suppressing the excessive ultraviolet light irradiation that may adversely affect plants and other objects located in the space.

Since the inactivation device in the configuration emits ultraviolet light even in the case of the reduced light intensity, it has been confirmed that the inactivation treatment is effective against the airborne bacteria present in the space around the inactivation device although the ultraviolet light hardly reaches the walls and floors. Specifically, irradiating human coronaviruses floating in the space with ultraviolet light having a wavelength of 222 nm is confirmed to achieve an inactivation effect of approximately 1 Log at an irradiation amount of ultraviolet light of 0.56 mJ.

Meanwhile, low-pressure mercury vapor lamps that emit ultraviolet light having a wavelength of 254 nm, which conventionally have been the mainstream germicidal lamps, has a difficulty in changing the intensity of the emitted ultraviolet light while keeping the lamp lit. For this reason, the method of changing the intensity of the emitted ultraviolet light for the inactivation treatment by ultraviolet light has been insufficiently studied so far.

From the above viewpoint, it is preferable that ultraviolet light has a main emission wavelength band including the peak wavelength in a range from 190 nm or more to less than 240 nm. Here, the main emission wavelength band refers to a wavelength that indicates an emission intensity of 50% or more with respect to the emission intensity of the peak wavelength; more preferably, an emission intensity of 30% or more; and especially preferably, an emission intensity of 10% or more.

In order to increase the utilization efficiency of ultraviolet light while enhancing the safety thereof, the main emission wavelength band including the peak wavelength is preferably in a range from 190 nm or more to 237 nm or less, more preferably in a range from 190 nm or more to 235 nm or less, and especially preferably in a range from 190 nm or more to 230 nm or less.

In addition, in order to suppress ozone generation more effectively, the main emission wavelength band including the peak wavelength is preferably in a range from 200 nm or more to 237 nm or less, more preferably in a range from 200 nm or more to 235 nm or less, and further more preferably in a range from 200 nm or more to 230 nm or less.

In the above inactivation device, the controller may be configured to control, in the main operation mode, the light source section in a manner that time for continuing the second lighting operation is longer than time for continuing the first lighting operation immediately before.

In addition, in the above inactivation device, the controller may be configured to control the light source section in a manner that the first lighting operation and the second lighting operation are periodically performed in the main operation mode.

In the present specification, "periodically" means that the first lighting operation and the second lighting operation are repeated with a preset operating time. It is noted that the variation of the time for the first lighting operation and the second lighting operation is allowed within a range that may be caused by the timer installed, circuit configuration, etc.

In addition, in the main operation mode, the period of switching between the first lighting operation and the second lighting operation does not always have to be constant; the period may be changed, for example, when the presence of a person is detected in the space or when a predetermined time has elapsed.

The above configuration suppresses the irradiation amount of ultraviolet light to a person when the inactivation treatment is performed in a space where a person is present, compared to the case in which the first lighting operation is always executed. Therefore, an inactivation device capable of safely inactivating pathogens in a space is achieved.

In addition, the above configuration further suppresses the power consumption and the excessive irradiation of ultraviolet light that may adversely affect plants and other objects located in the space when the inactivation treatment is performed in a space where no person is present, compared to the case in which the first lighting operation is always executed.

The controller of the above-mentioned inactivation device may be configured to execute, in the main operation mode, a control including a first control pattern that alternately performs the first lighting operation and an unlit operation, and a second control pattern that alternately performs the second lighting operation and an unlit operation, or that continuously performs the second lighting operation.

In addition, the above inactivation device may include a human detector that detects whether or not a person is present in a predetermined area; and the controller may be configured to execute the first control pattern when the human detector detects that no person is present, and execute the second control pattern when the human detector detects that a person is present.

The above configuration allows the inactivation device to be configured to execute the first control pattern that executes the first lighting operation, for example, in a case in which whether or not a person is present in a space or at a time of day when no person is expected to be present.

Providing the human detector such as a human detection sensor in the inactivation device for the above configuration enables a configuration of checking whether or not a person is present in the space and switching the control pattern.

It is noted that the inactivation device may adopt a configuration of switching a control pattern other than the configuration on the human detector. For example, the inactivation device may adopt a configuration of providing a timer in the inactivation device and switching from the second control pattern to the first control pattern at a time of day (typically, late at night) when no person is expected to be present.

In addition, the controller of the above-mentioned inactivation device may be configured to execute, in the main operation mode, a control including a third control pattern that alternately performs the first lighting operation and the second lighting operation, and a fourth control pattern that alternately performs the first lighting operation and the second lighting operation and that has a higher frequency of the second lighting operation per unit time compared to the third control pattern.

In addition, the above inactivation device may include a human detector that detects whether or not a person is present in a predetermined area, and the controller may be configured to execute the third control pattern when the human detector detects that no person is present, and to execute the fourth control pattern when the human detector detects that a person is present.

In the above inactivation device, the controller may be configured to shift from the main operation mode to a secondary operation mode different from the main operation mode upon a detection of satisfying a predetermined condition.

The term "secondary operation mode" here refers to a mode in which the lighting control of the light source section is performed in a control pattern different from that of the main operation mode in response to the number of persons present in the space, the time of day, the state of opening of windows and doors, etc., in addition to a lighting operation performed temporarily for the purpose of ensuring safety in the event of an abnormality occurred in the inactivation device.

In the inactivation treatment, it is expected to have cases in which the inactivation treatment always performing in a predetermined mode is undesirable. Examples include a case in which the inactivation treatment is desired to continue at least in the vicinity of the device although a person with a constitution that exhibits a hypersensitivity reaction to ultraviolet light or a person who dislikes being exposed to ultraviolet light enters a room. In addition, examples include a case in which the optimal operation mode is requested in response to the conditions of the environment in which the inactivation treatment is performed (e.g., degree of persons traffic, time of day or night).

Hence, the above configuration enables the temporal shift from the main operation mode to the secondary operation mode in the case of receiving a signal of refusing ultraviolet light irradiation as an example of a predetermined condition, and the execution of different operations or temporary operations in the case that the main operation mode is difficult to continue. The temporary operations may include the irradiation of ultraviolet light at a very low intensity, or the irradiation of ultraviolet light once an hour for a few seconds.

Examples of a predetermined condition include the case of receiving a wireless signal that refuses the ultraviolet light irradiation and the case of detecting the presence of a person or an animal within a few centimeters from the inactivation device.

The secondary operation mode may also be a mode determined by the controller in accordance with the conditions of the device itself to shift. For example, it can be a mode of executing the lighting control such that the light intensity is temporarily increased when an excimer lamp, LEDs, or other element mounted as a light source section deteriorates and the intensity of the emitted ultraviolet light falls below a predetermined value.

A method of inactivation treatment for bacteria and/or viruses according to the present invention is a method of inactivating bacteria and/or viruses present in a space, and includes a main treatment process including a first lighting operation in which an area to be treated is irradiated with ultraviolet light having a peak wavelength in a range from 190 nm or more to less than 240 nm, and a second lighting operation in which the area to be treated is irradiated with ultraviolet light having a peak wavelength in a range from 190 nm or more to less than 240 nm, and whose light intensity is lower than that of the first lighting operation.

In the above-mentioned method of inactivation treatment, the main treatment process may be performed by a light source emitting ultraviolet light mounted in a ceiling or an upper area of the space.

"Upper area of a space" in the present specification refers to an area whose height from a floor is higher than the height of a person; specifically, it refers to an area whose height from a floor is 2 m or higher. Methods of mounting inactivation devices in the upper area of the space include mounting them on a wall in the upper area of the space or fixing them on a pole having a height of 2 meters or more.

The illuminance of the ultraviolet light emitted from a light source is inversely proportional to the square of the separation distance from the light source section. Hence, it is desirable to maintain a certain separation distance between the light source and the person as much as possible so that the person is not irradiated with ultraviolet light immediately after it is emitted from the light source section.

If the light source is mounted on a floor or tabletop in a space, a person may approach the light source itself, and the irradiation amount may quickly reach the threshold limitvalue of the above-mentioned standard.

In contrast, the above configuration allows, for most persons, a separation distance of several tens of centimeters between their heads and the ceiling, even if they are in an upright position. In other words, there is no risk for a person to be unexpectedly exposed to high-illuminance ultraviolet light unless the person intentionally reaches toward the light source, for example.

Therefore, the above-mentioned method is capable of performing the inactivation treatment more safely in a space compared to the case in which the light source is mounted on a floor or tabletop.

In addition, in the above-mentioned method of inactivation treatment, the main treatment process may be a process that periodically performs the first lighting operation and the second lighting operation.

In the above-mentioned method of inactivation treatment, the main treatment process may include a process in which the time for continuing the second lighting operation is longer than time for continuing the first lighting operation immediately before.

In the above-mentioned main treatment process, the first lighting operation and the second lighting operation may be periodically performed.

In the above-mentioned inactivation treatment process, the main treatment process may include a first treatment process that alternately performs the first lighting operation and an unlit operation, and a second treatment process that alternately performs the second lighting operation and an unlit operation or that continuously performs the second lighting operation.

In the above inactivation treatment process, the main treatment process may include a third treatment process that alternately performs the first lighting operation and the second lighting operation, and a fourth treatment process that alternately performs the second lighting operation and the first lighting operation, and that has a higher frequency of the second lighting operation per unit time compared to the third treatment process.

The above-mentioned method of inactivation treatment may include a secondary treatment process that performs ultraviolet light irradiation different from that of the main treatment process upon a detection of satisfying a predetermined condition.

### Effects of the Invention

The present invention is capable of providing an inactivation device and a method of inactivation treatment for efficiently inactivating bacteria and/or viruses present in a space while ensuring safety for humans and animals.

### BRIEF DESCRIPTION OF DRAWINGS

FIG. 1A is a drawing schematically illustrating an embodiment of an inactivation device.
FIG. 1B is a drawing schematically illustrating an embodiment of an inactivation device.
FIG. 2 is a drawing of the inactivation device in FIG. 1A when viewed from the -Z side.
FIG. 3 is a cross-sectional view of the inactivation device in FIG. 1A when viewed in the X direction.
FIG. 4 is a cross-sectional view of the inactivation device in FIG. 1A when viewed in the Y direction.
FIG. 5 is a timing chart illustrating the change in the light intensity of ultraviolet light in the vicinity of a light emission window.
FIG. 6 is a timing chart illustrating the change in the light intensity of ultraviolet light in the vicinity of a light emission window.
FIG. 7 is a timing chart illustrating the change in the light intensity of ultraviolet light in the vicinity of a light emission window.
FIG. 8 is a timing chart illustrating the change in the light intensity of ultraviolet light in the vicinity of a light emission window.
FIG. 9 is a timing chart illustrating the change in the light intensity of ultraviolet light in the vicinity of a light emission window.
FIG. 10 is a timing chart illustrating the change in the light intensity of ultraviolet light in the vicinity of the light emission window.
FIG. 11 is a graph illustrating the absorbance characteristics of proteins in the ultraviolet light band.
FIG. 12 is a graph of comparison results between continuous lighting and intermittent lighting using a light source emitting ultraviolet light having a central wavelength of 254 nm.
FIG. 13 is a graph of comparison results between continuous lighting and intermittent lighting using a light source emitting ultraviolet light having a central wavelength of 222 nm.
FIG. 14 is a graph of comparison results between continuous lighting and intermittent lighting using a light source emitting ultraviolet light having a central wavelength of 207 nm.

### DESCRIPTION OF EMBODIMENTS

Hereinafter, an inactivation device and a method of inactivation treatment for bacteria and/or viruses according to the present invention will be described with reference to the drawings. Each of the following drawings is illustrated schematically, and the dimensional ratios and numbers in the drawings do not necessarily correspond to the actual dimensional ratios and numbers.

### Inactivation device

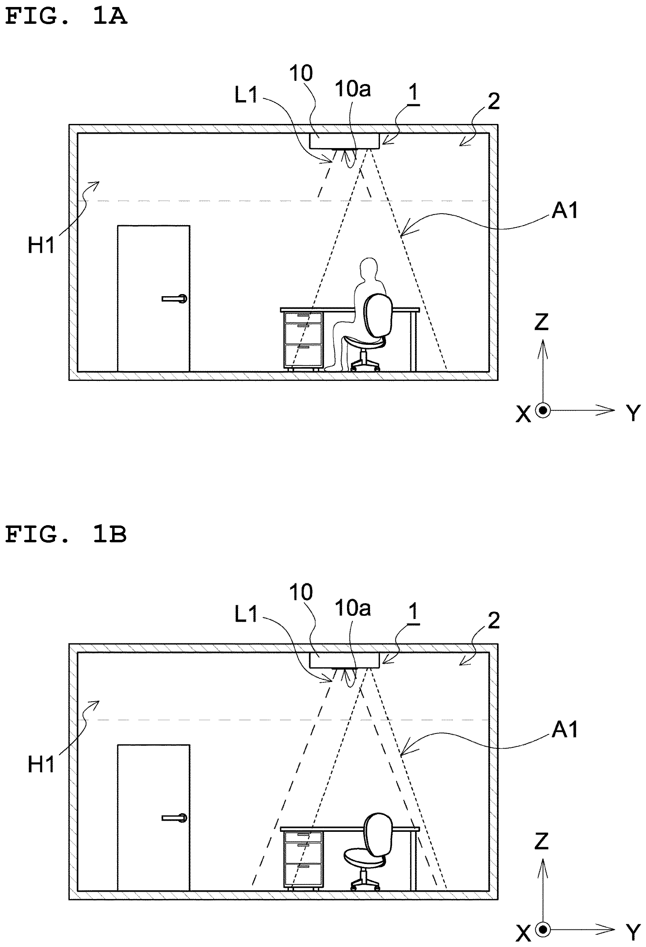

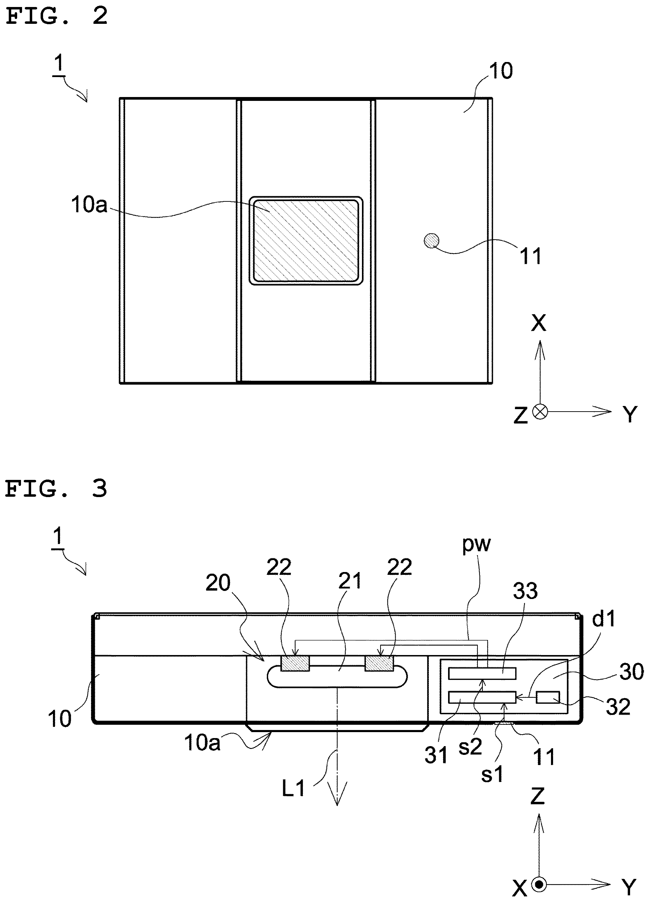

FIGS. 1A and 1B are drawings schematically illustrating an embodiment of an inactivation device. FIG. 1A illustrates the state of the inactivation treatment when a human detection sensor 11 (see FIG. 2), which will be described below, detects a person. FIG. B illustrates the state of the inactivation treatment when no person is detected. As shown in FIGS. 1A and 1B, the inactivation device 1 of the present embodiment is mounted on the ceiling to emit ultraviolet light L1 toward the floor surface in a space 2. This arrangement allows ultraviolet light L1 to be emitted from the upper area toward the lower area.

As will be described in detail in the explanation of the control method, FIG. 1A illustrates the state of a second lighting operation in which the ultraviolet light L1 with relatively low light intensity is emitted, which is a state of mainly inactivating pathogens floating in the space 2. FIG. 1B illustrates the state of a first lighting operation in which the ultraviolet light L1 with relatively high light intensity is emitted, which is a state of mainly inactivating pathogens adhering to objects such as desks and floors in addition to inactivating pathogens floating in the space 2.

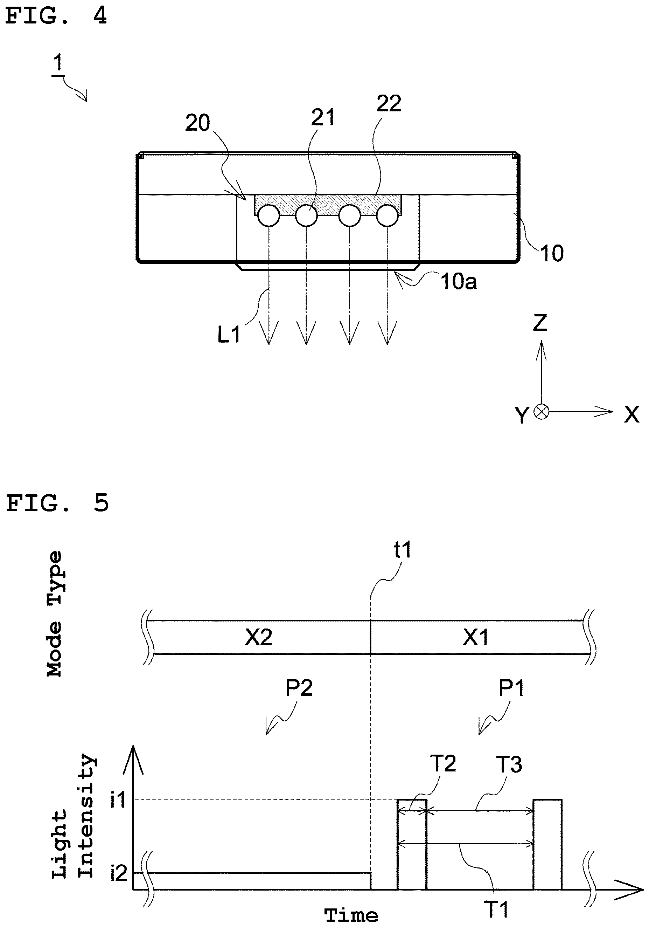

FIG. 2 is a drawing of the inactivation device 1 in FIG. 1A when viewed from the -Z side, FIG. 3 is a cross-sectional view of the inactivation device 1 in FIG. 1A when viewed in the X direction, and FIG. 4 is a cross-sectional view of the inactivation device in FIG. 1A when viewed in the Y direction. As shown in FIG. 2, the inactivation device 1 in the present embodiment includes an enclosure 10 formed with a light emission window 10a thereon. As shown in FIG. 3, the enclosure 10 includes a light source section 20 and a controller 30 thereinside.

The inactivation device 1 of the present embodiment will be described in a configuration in which it is mounted on the ceiling; however, the inactivation device 1 of the present invention may be mounted on a floor or a desk, or may be fixed even to a pole, for example, and used in an upper area H1 (see FIG. 1A) of the space 2. When the inactivation device 1 is fixed to a pole, for example, the inactivation device is preferably mounted at a height of 2 m or more above the floor in the space 2.

In the following description, the direction in which the ultraviolet light L1 is emitted is referred to as the Z direction, and the direction of the tube axis of the tube body 21 of the excimer lamp, which will be described later, is referred to the Y direction, as shown in FIG. 3. The direction orthogonal to the Y direction and the Z direction is referred to the X direction.

When a direction is expressed with distinguishing a positive direction from a negative direction, it is described with a positive or negative sign such as "+Z direction" or "-Z direction", and when a direction is expressed without distinguishing a positive direction from a negative direction, it is simply described as "Z direction".

As shown in FIG. 2, the enclosure 10 includes the light emission window 10a through which the ultraviolet light L1 is emitted outside and the human detection sensor 11 corresponding to a human detector on the surface where the light emission window 10a is formed.

The light emission window 10a is provided on the wall surface parallel to the XY-plane and is formed with a material that exhibits transparency with respect to the ultraviolet light L1 emitted from the light source section 20, which will be described below. Specifically, examples of the material constituting the light emission window 10a include quartz glass and sapphire glass. The light emission window 10a can also be an opening.

The light emission window 10a according to the present embodiment is preferably configured to suppress the light intensity in the wavelength range of 240 nm to 280 nm in order to enhance safety by suppressing the adverse effects on human bodies. As an example of the specific configuration, the light emission window 10a in the present embodiment is provided with an optical filter, which is not shown in the figure. However, an optical filter may not be provided when the light intensity in the wavelength band of 240 nm to 280 nm is sufficiently low, in the spectrum of the ultraviolet light L1 emitted from the light source section 20. The configuration of suppressing the light intensity in the above wavelength range can be achieved by a mechanism or an optical system provided separately from the enclosure 10 or the light emission window 10a in addition to the optical filter provided in the light emission window 10a.

Furthermore, the inactivation device 1 of the present embodiment is provided with the human detection sensor 11 that uses infrared light to detect a person on the side face of the enclosure 10. FIGS. 1A and 1B illustrate an area A1 where the human detection sensor 11 can receive infrared light, in other words, it indicates an area where the human detection sensor 11 can detect a person.

The human detection sensor 11 in the present embodiment is an infrared sensor, but can also adopt a sensor that detects the presence of a person, for example, a proximity sensor or a distance sensor. The inactivation device 1 may employ a sensor such as those described above as the human detection sensor 11 and may be configured to detect the presence of an animal and a predetermined object as well as the presence of a person.

As shown in FIG. 3, the light source section 20 in the present embodiment includes an excimer lamp that includes a tube body 21 and two electrodes 22 disposed on the outer side face of the tube body 21.

The tube body 21 is filled with krypton (Kr) and chlorine (CI) as luminescent gases. When a voltage equal to or greater than a predetermined threshold is applied between the electrodes 22, ultraviolet light L1 having a peak wavelength of 222 nm is emitted from the tube body 21. The light source section 20 in the first embodiment is constituted by an excimer lamp; however, it can be constituted by any light source capable of emitting ultraviolet light L1 in the wavelength band described above that can be used for the inactivation treatment for pathogens, for example, LEDs.

As shown in FIGS. 1A and 1B, the ultraviolet light L1, which is emitted from the light source section 20, is emitted through the light emission window 10a to the outside of the enclosure 10. As shown in FIGS. 1A and 1B, the inactivation device 1 is mounted on the ceiling of the space 2 and radiates the ultraviolet light L1 toward the floor (-Z side), which is a target area for treatment. The walls in the space 2 may be a target area for treatment, and the target area for treatment may also include the top of a desk mounted in the space 2.

As shown in FIG. 3, the controller 30 in the present embodiment includes an arithmetic unit 31 that calculates the value of power pvv to be supplied to the light source section 20, a memory unit 32, which stores data d1 of the control pattern, and a drive unit 33 that supplies the power pvv to the light source section 20.

The arithmetic unit 31 in the present embodiment is an arithmetic circuit mounted in the enclosure 10, and examples thereof include a CPU or an MPU. The drive unit 33 is an electric circuit that generates power pvv to drive the light source section 20. The memory unit 32 can adopt memory devices such as flash memory and semiconductor memory; however, the memory unit 32 is preferably nonvolatile memory from the viewpoint in which the process of storing the data d1 for the control pattern is unnecessary each time the power is turned on. The memory unit 32 can also be an external memory connected directly or via a cable or the like.

When receiving a signal s1 from the human detection sensor 11 that a person has been detected or is no longer detected, the arithmetic unit 31 reads the data d1 from the memory unit 32, the data d1 being associated with the control pattern in accordance with the s1. When reading the data d1 from the memory unit 32, the arithmetic unit 31 outputs, based on the data d1, a signal s2 to the drive unit 33, the signal s2 specifying the value of the power pvv to be supplied to the light source section 20.

The drive unit 33 generates the power pvv to be supplied to the light source section 20 based on the signal s2, and supplies the power pvv to the electrode 22 provided in the light source section 20.

The memory unit 32 in the present embodiment stores the data d1 of the plurality of control patterns. These specific controls will be described below.

The controller 30 in the present embodiment is described as a configuration in which it is mounted in the enclosure; however, the controller 30 can be an external device such as a PC or a tablet that is configured to communicate with the inactivation device 1 in a wired or wireless manner.

### Control method

Hereinafter, the control of the light source section 20 with the controller 30 will be described in detail. The control pattern described below is merely an expected example based on the idea of the method of inactivation treatment in the present invention. The controller 30 in the present embodiment can be expected to automatically control the light source section 20.

The inactivation device 1 in the present embodiment is configured to perform an operation pattern including the inactivation treatment operation that includes the first lighting operation in which the ultraviolet light L1 is emitted with relatively high light intensity and the second lighting operation that in which the ultraviolet light L1 is emitted with relatively low light intensity.

As shown in FIG. 1B, the first lighting operation, which emits the ultraviolet light L1 with relatively high light intensity, is an operation of irradiating a desk or a floor with the ultraviolet light L1 and is mainly performed for the inactivation treatment for pathogens adhering to the floor and the upper surface of the desk as well as for the inactivation treatment for pathogens floating in the space 2.

As shown in FIG. 1A, the second lighting operation, which emits ultraviolet light L1 with relatively low light intensity, is mainly performed for the inactivation treatment for pathogens floating in the space 2 while suppressing the irradiation of the ultraviolet light L1 with a person.

Hereinafter, examples of the inactivation treatment with a combination of the first lighting operation and the second lighting operation will be described. Each of these examples is a control method of efficiently performing the inactivation treatment for pathogens present in the space 2 by combining treatments in which the light intensity of the ultraviolet light L1 emitted from the inactivation device 1 differs.

Examples of the control method include a control method having a control pattern of switching between the first lighting operation and the second lighting operation depending on whether or not a person is present in the space 2 using a human detector (human detection sensor 11 in the present embodiment), which detects whether or not a person is present in the predetermined area. Examples of the control method also include a control method having a control pattern of alternately switching between the first lighting operation and the second lighting operation during a period when the human detector fails to detect a person. Furthermore, examples of the control method include a control method having a control pattern of alternately switching between the first lighting operation and the second lighting operation during a period when the human detector detects a person.

Furthermore, the control method may employ a control pattern that appropriately combines a control pattern (first control pattern) periodically repeating the first lighting operation and turning off the light with a control pattern (second control pattern) periodically repeating the second lighting operation and turning off the light. The switching of the control pattern may adopt a control method of switching the control pattern depending on whether or not a person is present in the space 2 or a control method of alternately switching the first control pattern and the second control pattern during the period when the human detector detects a person or during the period when the human detector fails to detect a person. Hereinafter, examples of various possible control methods will be presented.

In the embodiment of maintaining the lighting state by performing the first lighting operation and the second lighting operation without turning off the light in the middle of the operation, the inactivation treatment for pathogens floating in the space 2 continues, thus the embodiment effectively performs the inactivation treatment for pathogens, compared to the method of performing the lighting operation and the unlit operation.

Specifically, in the case of the method of repeating the lighting operation and the unlit operation, when pathogens flow in sequentially from outside the space 2, the inactivation treatment for pathogens floating in the space 2 may not be enough, even though the inactivation treatment at the area irradiated with the ultraviolet light L1 is sufficiently operated. In such a case, the control method of maintaining the lighting and continuing the inactivation treatment for pathogens floating in the space 2 can inactivate pathogens that sequentially flow into the space 2 at any time, maintaining a sanitary environment.

The following examples describe the main operation mode, which is a normal operation in which the first lighting operation and the second lighting operation are performed; however, the inactivation device 1 may be configured to shift to a secondary operation mode in which the control pattern and operation are different from those of the main operation mode when predetermined conditions are met, such as a decrease in the output of the light source section 20.

Examples of the conditions on shifting to the secondary operation mode include the case in which a decrease in the intensity of ultraviolet light L1 emitted from the light source section 20 is detected due to the degradation of the light source section 20. In such a case, the controller 30 shifts to the secondary operation mode and executes the inactivation treatment by increasing the value of the power pvv supplied from the drive unit 33, or by stopping supplying power pvv to the light source section 20.

### Example 1

FIG. 5 is a timing chart illustrating the changes in the light intensity of ultraviolet light L1 in the vicinity of the light emission window 10a in Example 1. FIG. 5 illustrates a mode type regarding whether or not a person is detected and a timing chart regarding light intensity. The mode type in FIG. 5 shows the period during which the human detection sensor 11 fails to detect a person as a first period X1, and the period during which the human detection sensor 11 detects a person as a second period X2. Example 1 describes the operation in which the human detection sensor 11 detects whether or not a person is present in the space 2 and switches the control pattern as the main operation mode.

The switching between the first period X1 and the second period X2 can be employed by conditions other than human detection with the human detection sensor 11. Examples of conditions switching between the first period X1 and the second period X2 may include the case in which a timer detects that a predetermined time has elapsed or a predetermined time has arrived.

In the timing chart of FIG. 5, the vertical axis represents the light intensity, and the section of the light intensity with a relatively high level (i1) corresponds to an execution period of the control pattern P1 and the section of the light intensity with a relatively low level (i2) corresponds to an execution period of the control pattern P2.

In the present embodiment, the controller 30 is configured to switch to the control pattern P2 when the human detection sensor 11 detects the presence of a person, and the controller 30 is configured to execute the control pattern P1 when the human detection sensor no longer detects the presence of a person. The timing chart shown in FIG. 5 illustrates the state around a time t1 when the human detection sensor 11 no longer detects the presence of a person and switches from the state in which the controller executes the control pattern P2 to the state in which the controller executes the control pattern P1.

The control pattern P1 in Example 1 is a pattern in which the controller 30 controls the light source section 20 to emit the ultraviolet light L1 with a predetermined intensity in order to perform the inactivation treatment for pathogens adhering to the surface of objects such as desks, floors, as well as the inactivation treatment for pathogens floating in the space 2. The control pattern P1 is a pattern in which the first lighting operation of lighting with a predetermined light intensity and the unlit operation are periodically repeated, and corresponds to the first control pattern (first treatment process).

In Example 1, in the control pattern P1 shown in FIG. 5, a period T1 for repeating the first lighting operation and the unlit operation is set to 45 seconds, a time T2 for continuing the first lighting operation is set to 15 seconds, and a time T3 for continuing the unlit operation is set to 30 seconds.

The control pattern P2 in Example 1 is a mode in which the intensity of the ultraviolet light L1 emitted from the light source section 20 is reduced when a person is present in the space 2 in order to mainly perform inactivation treatment for pathogens floating in the space 2 while suppressing the irradiation amount of the ultraviolet light L1 radiated to the person. The control pattern P2 is a pattern that performs the second lighting operation in which the light intensity thereof is lower than that of the first lighting operation in the control pattern P1, and corresponds to the second control pattern (second treatment process).

In the control pattern P2 in Example 1, as shown in FIG. 5, the controller 30 continuously supplies the light source section 20 with the power pvv required for the second lighting operation and continuously performs the second lighting operation.

Executing the above control method allows the inactivation device 1 to perform the control pattern P2 when a person is present in the space 2, providing the inactivation treatment with the second lighting operation, which has a lower light intensity than that of the first lighting operation. Hence, the irradiation amount of the ultraviolet light L1 radiated to a person is reduced when the person is present in the space 2 compared to the case of always performing the first lighting operation.

In addition, the above control method eliminates the need for the continuous irradiation of the ultraviolet light L1 with high intensity when no person is present in the space 2, which leads to, for example, reducing the power consumption and suppressing the excessive irradiation of the ultraviolet light L1 that may affect objects placed in the space 2 such as plants.

In particular, since pathogens adhering to the surfaces of objects such as desks and floors remain in place, even the control method such as the control pattern P1 in which the first lighting operation of lighting with a predetermined light intensity is performed intermittently can effectively perform the inactivation treatment. In particular, ultraviolet light with a wavelength of less than 240 nm is unlikely to decrease the effects of the inactivation treatment even in the intermittent lighting operation because it has been found to have the effects of inhibiting the photoreactivation of the bacteria.

Therefore, the inactivation device 1 is capable of efficiently performing the inactivation treatment for pathogens present in the space 2 while ensuring safety for people and animals.

Furthermore, ultraviolet light with a wavelength of less than 240 nm is unlikely to decrease the effects of inactivation treatment even under visible light irradiation because it has been found to have the effects of inhibiting the photoreactivation of bacteria.

The inactivation device 1 in the present embodiment is configured to execute only the main operation mode (main treatment process) including the first lighting operation and the second lighting operation. The operations described below are all described as the control pattern of the main operation mode. However, in the inactivation device 1 of the present embodiment, controller 30 may be configured to shift to the secondary operation mode (secondary treatment process) that is different from the main operation mode when predetermined conditions are met. The secondary operation mode has been described above.

### Example 2

The control method of Example 2 of the inactivation device 1 of the present invention will now be described, focusing on the points that differ from those of Example 1.

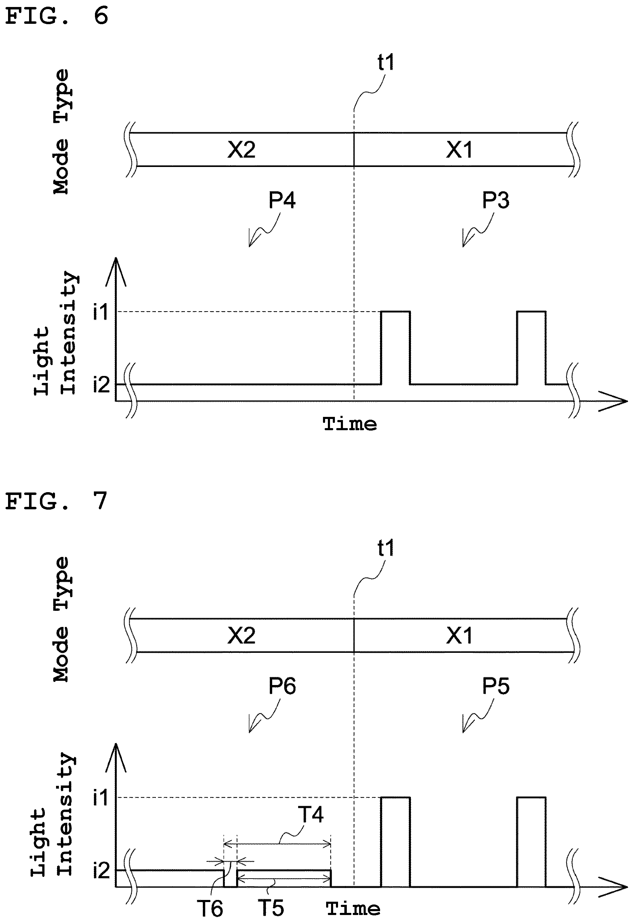

FIG. 6 is a timing chart illustrating the changes in the light intensity of ultraviolet light L1 in the vicinity of the light emission window 10a in Example 2. FIG. 6 illustrates a mode type regarding whether or not a person is detected and a timing chart regarding the light intensity. In the timing chart of FIG. 5, a vertical axis represents the light intensity, a section repeating the light intensity with a relatively high level (i1) and the light intensity with a relatively low level (i2) corresponds to an execution period of a control pattern P3, and a section of the light intensity with a relatively low level (i2) corresponds to an execution period of a control pattern P4. In Example 2, the execution period of the control pattern P3 and the execution period of the control pattern P4 are described as the main operation mode.

The control pattern P3 in Example 2 is similar to the control pattern P1 in Example 1; specifically, the controller 30 controls the light source section 20 to emit the ultraviolet light L1 with a predetermined intensity in order to perform the inactivation treatment for pathogens adhering to the floor of the space 2 as well as the inactivation treatment for pathogens floating in the space 2. As shown in FIG. 6, the control pattern P3 corresponds to a third control pattern (third treatment process) in which the first lighting operation and the second lighting operation are repeated periodically.

As shown in FIG. 6, the control pattern P4 in Example 2 corresponds to the second control pattern (second treatment process), in which the second lighting operation is continuously performed similarly to that in Example 1.

Executing the above control method allows the light source section 20 to reduce the time of day of being completely unlit and to continuously perform the inactivation treatment for pathogens floating in the space 2, thus maintaining the sanitary conditions in the space 2. In addition, reducing the time of day of being unlit lowers the concern of discontinuing the inactivation treatment due to the failure of turning on again.

### Example 3

The control method of Example 3 of the inactivation device 1 of the present invention will now be described, focusing on the points that differ from those of Example 1 and Example 2.

FIG. 7 is a timing chart illustrating the changes in the light intensity of ultraviolet light L1 in the vicinity of the light emission window 10a in Example 3. FIG. 7 illustrates a mode type regarding whether or not a person is detected and a timing chart regarding the light intensity. In the timing chart of FIG. 7, a vertical axis represents the light intensity, a section of the light intensity with a relatively high level (i1) corresponds to an execution period of a control pattern P5, and a section of the light intensity with a relatively low level (i2) corresponds to an execution period of a control pattern P6. In Example 3, the execution period of the control pattern P5 and the execution period of the control pattern P6 are described as the main operation mode.

As shown in FIG. 7, the control pattern P5 in Example 3 corresponds to the first control pattern (first treatment process), in which the controller 30 periodically repeats the first lighting operation and the unlit operation as is similar to the control pattern P1 in Example 1.

As shown in FIG. 7, the control pattern P6 in Example 3 corresponds to the second control pattern (second treatment process), in which the second lighting operation and the unlit operation are repeated periodically.

In Example 3, in the control pattern P6 shown in FIG. 7, a period T4 for repeating the second lighting operation and the unlit operation is set to 60 seconds, a time T5 for continuing the second lighting operation is set to 50 seconds, and a time T6 for continuing the unlit operation is set to 10 seconds.

Executing the above control method allows the light source section 20 to reduce the overall lighting time, thus extending the service life of the light source constituting the light source section 20, compared to those of the above-mentioned Example 1 and Example 2.

### Example 4

The control method of Example 4 of the inactivation device 1 of the present invention will now be described, focusing on the points that differ from those of Example 1, Example 2, and Example 3.

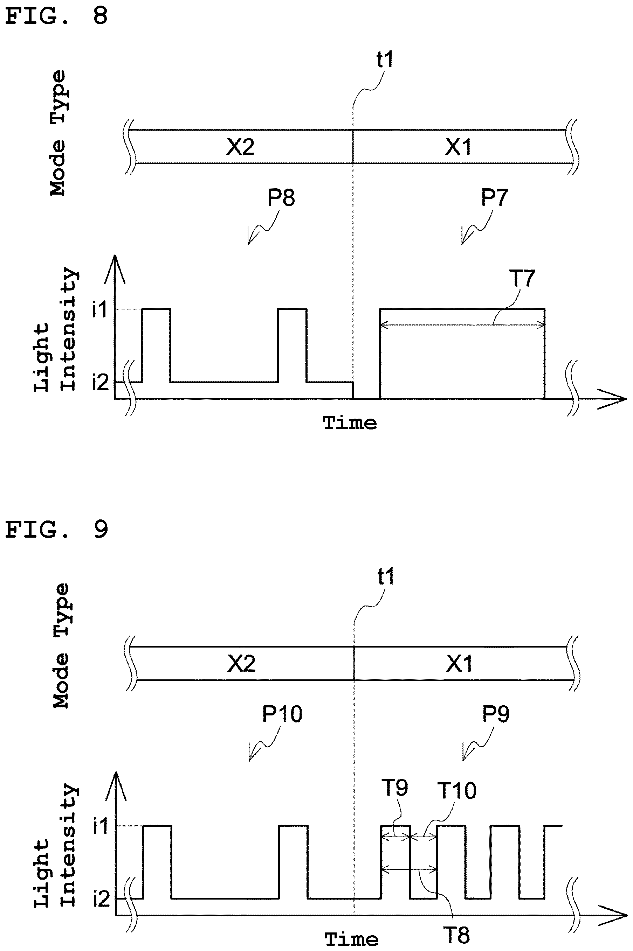

FIG. 8 is a timing chart illustrating the changes in the light intensity of ultraviolet light L1 in the vicinity of the light emission window 10a in Example 4. FIG. 8 illustrates a mode type regarding whether or not a person is detected and a timing chart regarding the light intensity. In the timing chart of FIG. 8, a vertical axis represents the light intensity, a section of the light intensity with a relatively high level (i1) corresponds to an execution period of a control pattern P7, and a section of repeating the light intensity with a relatively high level (i1) and the light intensity with a relatively low level (i2) corresponds to an execution period of a control pattern P8. In Example 4, the execution period of the control pattern P8 is described as the main operation mode; however, the execution period of the control pattern P7 and the execution period of the control pattern P8 may be regarded as the main operation mode.

As shown in FIG. 8, the control pattern P7 in Example 4 corresponds to the second control pattern (second treatment process), in which the second lighting operation and the unlit operation are repeated periodically. It is noted that the control pattern P8 in Example 4 is a pattern of maintaining the lighting state of the first lighting operation for a longer time than that in Example 1.

As shown in FIG. 8, the control pattern P8 in Example 4 corresponds to the third control pattern (third treatment process), in which the controller 30 periodically repeats the first lighting operation and the second operation as is similar to the control pattern P3 in Example 2.

In Example 4, in the control pattern P7 shown in FIG. 8, a time T7 for continuing the first lighting operation is set to 600 seconds.

Executing the above-mentioned control method allows a longer irradiation time of the ultraviolet light L1 with high intensity to the space 2 while suppressing the irradiation amount of the ultraviolet light L1 radiated to a person, achieving higher inactivation effects compared to Examples 1 to 3.

Furthermore, in the control pattern P7, the longer irradiation of the ultraviolet light L1 with high intensity can inactivate pathogens adhering to the surfaces of objects to the required level in a shorter time. After a sufficient irradiation time for inactivation treatment has elapsed, the light may be turned off as shown in FIG. 8.

### Example 5

The control method of Example 5 of the inactivation device 1 of the present invention will now be described, focusing on the points that differ from those of Examples 1 to 4.

FIG. 9 is a timing chart illustrating the changes in the light intensity of ultraviolet light L1 in the vicinity of the light emission window 10a in Example 5. FIG. 9 illustrates a mode type regarding whether or not a person is detected and a timing chart regarding the light intensity. In the timing chart of FIG. 9, a vertical axis represents the light intensity.

As shown in FIG. 9, Example 5 is a pattern of periodically repeating the light intensity with a relatively high level (i1) and the light intensity with a relatively low level (i2). A period of high frequency per unit time of the light intensity with a relatively low level (i2) corresponds to an execution period of a control pattern P9, and a period of low frequency per unit time of the light intensity with a relatively low level (i2) corresponds to an execution period of a control pattern P10. In other words, the frequency per unit time of the second lighting operation is higher in the control pattern P9 than in the control pattern P10, and the control pattern P9 corresponds to the third control pattern (third treatment process) and the control pattern P10 corresponds to the fourth control pattern (fourth treatment process). In Example 5, the execution period of the control pattern P9 is described as the main operation mode; however, the execution period of the control pattern P9 and the execution period of the control pattern P10 may be regarded as the main operation mode.

In Example 5, the control pattern P10 shown in FIG. 9 has the same time setting as that of the control pattern P3 in Example 1. In the control pattern P9, a period T8 for repeating the first lighting operation and the second operation is set to 30 seconds, a time T9 for continuing the first lighting operation is set to 15 seconds, and a time T10 for continuing the second operation is set to 15 seconds.

Executing the above-mentioned control method allows a longer irradiation time of the ultraviolet light L1 with high intensity to the space 2 while suppressing the irradiation amount of the ultraviolet light L1 radiated to a person, achieving higher inactivation effects compared to Examples 1 to 3. Furthermore, the light source section 20 is controlled to reduce the time of day of being completely unlit, and to continuously perform the inactivation treatment for pathogens floating in the space 2, thus maintaining the sanitary conditions in the space 2. In addition, reducing the time of day of being unlit lowers the concern of discontinuing the inactivation treatment due to the failure of turning on again.

### Example 6

The control method of Example 6 of the inactivation device 1 of the present invention will now be described, focusing on the points that differ from those of Example 1 to 5.

FIG. 10 is a timing chart illustrating the changes in the light intensity of ultraviolet light L1 in the vicinity of the light emission window 10a in Example 6. FIG. 10 illustrates a mode type regarding whether or not a person is detected and a timing chart regarding the light intensity. In the timing chart of FIG. 10, a vertical axis represents the light intensity.

As shown in FIG. 10, Example 6 is a pattern of periodically repeating the light intensity with a relatively high level (i1) and the light intensity with a relatively low level (i2). The pattern remains unchanged on a time of maintaining the light intensity with a relatively low level (i1) and a time of maintaining the light intensity with a relatively low level (i2). For the sake of distinction, a period when the human detection sensor 11 fails to detect a person corresponds to an execution period of a control pattern P11, and a period when the human detection sensor 11 detects a person corresponds to an execution period of a control pattern P12. In Example 6, the execution period of the control pattern P11 is described as the main operation mode; however, the execution period of the control pattern P11 and the execution period of the control pattern P12 may be regarded as the main operation mode. Specifically, the main operation mode may be a control pattern that alternately switches between the first lighting operation and the second lighting operation during the period when the human detector (human detection sensor 11 in Example 6) fails to detect a person. The main operation mode may also be a control pattern that alternately switches between the first lighting operation and the second lighting operation during the period when the human detector detects a person.

Even the method in Example 6 suppresses the irradiation amount of ultraviolet light L1 radiated to a person compared to the control method of always continuing the first lighting operation. The method in Example 6 always continues the inactivation treatment for pathogens floating in the space 2, thus performing the inactivation treatment for pathogens more effectively compared to the method of performing the lighting operation and the unlit operation. In addition, eliminating the need for distinguishing whether or not a person is detected, the method in Example 6 does not always need a human detector that detects whether or not a person is present in a predetermined area.

Examples of the control methods have been described; however, each example is part of the examples that can be implemented by the present invention. The control method of the present invention is not limited to the methods described above. The configuration of the inactivation device 1 is not limited to the configurations described above.

Other specific examples may include a configuration of switching to a control pattern (the first control pattern or the third control pattern) that intensively performs the inactivation treatment only during midnight in a workplace space, and a configuration of switching to another control pattern (the second control pattern or the fourth control pattern) when the brightness falls below a predetermined level by a person turning off the room lights or closing the window curtains.

The above describes the control pattern of repeating the first lighting operation and the second lighting operation and the control pattern of repeating the lighting operation and the unlit operation as the control pattern of repeating each operation periodically; however, the switching of each operation may be configured to change gradually over time.

For example, the control pattern may be such that the first lighting operation is initially performed for a long period of time, and then the first lighting operation and the second lighting operation are repeated in response to the movement of pathogens by natural convection or the like. The control pattern may be configured to further include an operation of emitting the ultraviolet light L1 with a light intensity different from that of the first lighting operation and the second lighting operation.

In the above description, the control performed by the controller 30 is described as the main operation mode regardless of whether or not the human detection sensor 11 detects the presence of a person; however, the mode performed when the human detection sensor 11 detects the presence of a person may be the main operation mode.

The light intensity of the first lighting operation and the second lighting operation is set appropriately according to the installation conditions in which the inactivation device is used. For example, the appropriate difference in light intensity depends on the height of the ceiling on which the device is to be mounted. As an example, the light intensity of the ultraviolet light at the light emission window 10a in the second lighting operation may be controlled to be 80% or less of that in the first lighting operation. Also, the light intensity of the ultraviolet light at the light emission window 10a in the second lighting operation may be controlled to be 50% or less of that in the first lighting operation. Furthermore, the light intensity of the ultraviolet light at the light emission window 10a in the second lighting operation may be controlled to be 20% or less of that in the first lighting operation. Keeping the light intensity of the second lighting operation lower as described above allows the execution period of the main operation mode to be set for a longer period of time.

The present invention uses ultraviolet light having a peak wavelength within a range from 190nm or more to less than 240 nm for inactivating bacteria, fungi, viruses, and other pathogens present in the environment. As shown in FIG. 11, proteins are less likely to absorb ultraviolet light having a wavelength of 240 nm or more, and more likely to absorb ultraviolet light having a wavelength of 240 nm or less toward 200 nm. Ultraviolet light having a wavelength of 240 nm or more easily transmits human skin and penetrates the skin. Hence, cells inside human skin are prone to damage. In contrast, ultraviolet light having a wavelength of less than 240 nm is easily absorbed by the surface of human skin (e.g., stratum corneum) and is difficult to penetrate the skin. Hence, it is safe for the skin.

Furthermore, ultraviolet light having a wavelength of less than 240 nm can be expected to inhibit the "photoreactivation" of bacteria. The photoreactivation of bacteria results from the action of photoreactivation enzymes (e.g., FAD (flavin adenine dinucleotide)) possessed by the bacteria. Some bacteria can repair DNA damage when irradiated with light having a wavelength of 300 nm to 500 nm. For example, ultraviolet light having a wavelength of 254 nm, which is considered a conventional germicidal ray, damages the DNA of bacteria and viruses to inactivate them. However, under bright environments where visible light such as sunlight or white illumination radiates, the aforementioned photoreactivation enzymes act to repair DNA damage caused by ultraviolet light (photoreactivation), posing a problem that the inactivation is difficult to proceed. This problem is more pronounced in the case of performing unlit operations in which no ultraviolet light radiates or reduced light operations in which the light intensity of ultraviolet light is reduced, for example, the case in which ultraviolet light temporarily radiates and the case in which ultraviolet light intermittently radiates.

However, the absorptance for proteins is found to significantly increase in the wavelength band of shorter than 240 nm. Thus, light is effectively absorbed by proteins, which are components of cell membranes and enzymes of bacteria and viruses. In particular, bacteria and viruses are physically much smaller than human cells, thus ultraviolet light can easily reach their interior even if its wavelength band is shorter than 240 nm. In other words, ultraviolet light having a wavelength band shorter than 240 nm can inactivate microorganisms and viruses while suppressing their adverse effects on humans and animals. Furthermore, ultraviolet light having a wavelength band shorter than 240 nm can effectively act on the cells constituting bacteria and viruses, especially on cell membranes and enzymes containing protein components, enhancing the effects of suppressing functions such as the photoreactivation of bacteria.

The following are the verification in which continuous lighting and intermittent lighting are confirmed to provide a difference in the effects of the inactivation treatment for pathogens with different wavelengths of ultraviolet light.

Staphylococcus aureus, which has photoreactivation enzymes, was used as the bacteria to be inactivated. The changes in the survival rate of the bacteria were confirmed in the case of the continuous lighting of the above ultraviolet light and the case of the intermittent lighting of the above ultraviolet light under an environment where visible light was emitted.

The ultraviolet light sources used were a low-pressure mercury vapor lamp that emits ultraviolet light having a center wavelength of 254 nm, a KrCI excimer lamp that emits ultraviolet light having a center wavelength of 222 nm, and a KrBr excimer lamp that emits ultraviolet light having a center wavelength of 207 nm.

In the continuous lighting, the illuminance of the ultraviolet light radiated to the bacteria was set to 0.1 mW/cm².

The conditions for the intermittent lighting were set to a lighting time Ta = 50 (sec), an unlit time Tb = 59 minutes and 10 seconds (i.e., 3,550 (sec)), and a lighting duty ratio of 1.39 (%). The lighting duty ratio is defined as a ratio of the lighting time Ta to the total sum of the lighting time Ta and the unlit time Tb and is expressed by Td = Ta/(Ta + Tb). The illuminance of the ultraviolet light during the lighting in the intermittent lighting was set to 0.1 mW/cm², and the irradiation amount of ultraviolet light per lighting operation was set to 5 mJ/cm².

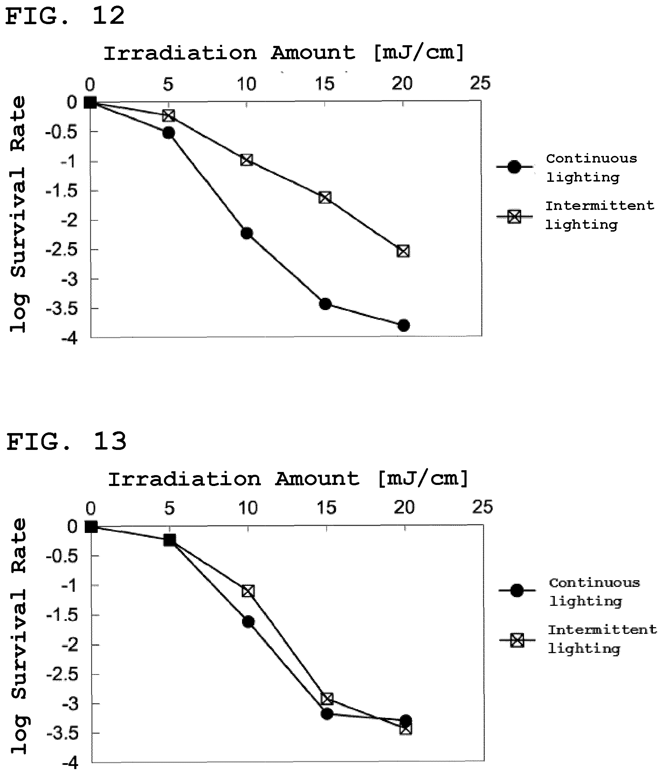

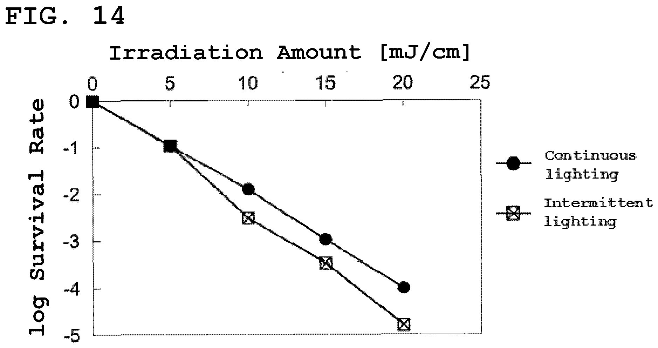

FIG. 12 is a chart exemplarily illustrating the results of the comparison between the continuous lighting and the intermittent lighting using ultraviolet light having a wavelength of 254 nm. FIG. 13 is a chart exemplarily illustrating the results of the comparison between the continuous lighting and the intermittent lighting using ultraviolet light having a wavelength of 222 nm. FIG. 14 is a chart exemplarily illustrating the results of comparison between the continuous lighting and the intermittent lighting using ultraviolet light having a wavelength of 207 nm. Referring to FIGS. 12 to 14, the horizontal axis denotes the irradiation amount of ultraviolet light (mJ/cm²) and the vertical axis denotes the log survival rate of the bacteria.

As shown in FIG. 12, in the case of ultraviolet light irradiation at a wavelength of 254 nm, the inactivation effect of intermittent lighting is inferior to that of continuous lighting. This is considered to be caused by the fact that the bacteria are photo-reactivated during the unlit time of the intermittent lighting. Hence, ultraviolet light irradiation at a wavelength of 254 nm with the intermittent lighting cannot ensure the inactivation of the bacteria because the bacteria exert a photoreactivation effect. In contrast, as shown in FIG. 13, in the case of ultraviolet light irradiation at a wavelength of 222 nm, the inactivation effect is equivalent between the intermittent lighting and the continuous lighting, because the photoreactivation of bacteria is inhibited.

As shown in FIG. 14, even in the case of ultraviolet light irradiation at a wavelength of 207 nm, the inactivation effect is found to be equivalent between the intermittent lighting and the continuous lighting. This is considered to be caused by the fact that the photoreactivation of the bacteria itself was inhibited by the ultraviolet light irradiation, which is similar to the case of the ultraviolet light irradiation at a wavelength of 222 nm. Each of the light sources uses a wavelength selective filter to cut off ultraviolet light having a wavelength from 240 nm or more to 300 nm or less, emitting ultraviolet light belonging to a wavelength band of 190 nm or more to less than 240 nm.

As described above, ultraviolet light having a wavelength band shorter than 240 nm inactivates microorganisms and viruses while suppressing adverse effects on humans and animals. Furthermore, it has been confirmed that the ultraviolet light effectively acts on cells constituting bacteria and viruses, especially on cell membranes containing protein components and enzymes, achieving effects in suppressing functions such as the photoreactivation of bacteria.

### Reference Signs List

- 1: Inactivation device
- 2: Space
- 10: Enclosure
- 10a: Light emission window
- 11: Human detection sensor
- 20: Light source section
- 21: Tube body
- 22: Electrode
- 30: Controller
- 31: Arithmetic unit
- 32: Memory unit
- 33: Drive unit
- A1: Area
- H1: Upper area
- L1: Ultraviolet light
- d1: Data
- s1: Signal
- s2: Signal
- pvv: Power
- X1: First period
- X2: Second period

## Claims

1. An inactivation device for bacteria and/or viruses in a space, comprising:
a light source section configured to emit ultraviolet light having a peak wavelength in a range from 190 nm or more to less than 240 nm, and
a controller configured to control the light source section to execute a main operation mode including a first lighting operation and a second lighting operation in which ultraviolet light is emitted with lower light intensity than that of the first lighting operation.

2. The inactivation device for bacteria and/or viruses according to claim 1, wherein the controller is configured to control, in the main operation mode, the light source section in a manner that a time for continuing the second lighting operation is longer than a time for continuing the first lighting operation immediately before.

3. The inactivation device for bacteria and/or viruses according to claim 1 or 2, wherein the controller is configured to control the light source section in a manner that the first lighting operation and the second lighting operation are periodically performed in the main operation mode.

4. The inactivation device for bacteria and/or viruses according to claim 1, wherein the controller is configured to execute, in the main operation mode, a control including a first control pattern that alternately performs the first lighting operation and an unlit operation, and a second control pattern that alternately performs the second lighting operation and an unlit operation, or that continuously performs the second lighting operation.

5. The inactivation device for bacteria and/or viruses according to claim 4, further comprising a human detector that detects whether or not a person is present in a predetermined area; and
wherein the controller is configured to execute the first control pattern when the human detector detects that no person is present, and execute the second control pattern when the human detector detects that a person is present.

6. The inactivation device for bacteria and/or viruses according to claim 1, wherein the controller is configured to execute, in the main operation mode, a control including a third control pattern that alternately performs the first lighting operation and the second lighting operation, and a fourth control pattern that alternately performs the first lighting operation and the second lighting operation and that has a higher frequency of the second lighting operation per unit time compared to the third control pattern.

7. The inactivation device for bacteria and/or viruses according to claim 6, further comprising a human detector that detects whether or not a person is present in a predetermined area, and
wherein the controller is configured to execute the third control pattern when the human detector detects that no person is present, and to execute the fourth control pattern when the human detector detects that a person is present.

8. The inactivation device for bacteria and/or viruses according to claim 1, wherein the controller is configured to shift from the main operation mode to a secondary operation mode different from the main operation mode upon a detection of satisfying a predetermined condition.

9. A method of inactivation treatment for bacteria and/or viruses in a space, comprising:
a main treatment process including a first lighting operation in which an area to be treated is irradiated with ultraviolet light having a peak wavelength in a range from 190 nm or more to less than 240 nm, and a second lighting operation in which the area to be treated is irradiated with ultraviolet light having a peak wavelength in a range from 190 nm or more to less than 240 nm, and whose light intensity is lower than that of the first lighting operation.

10. The method of inactivation treatment for bacteria and/or viruses according to claim 9, wherein the main treatment process is performed by a light source emitting ultraviolet light mounted in a ceiling or an upper area of the space.

11. The method of inactivation treatment for bacteria and/or viruses according to claim 9 or 10, wherein the main treatment process includes a process in which a time for continuing the second lighting operation is longer than a time for continuing the first lighting operation immediately before.

12. The method of inactivation treatment for bacteria and/or viruses according to claim 9 or 10, wherein the main treatment process is a process that periodically performs the first lighting operation and the second lighting operation.

13. The method of inactivation treatment for bacteria and/or viruses according to claim 9 or 10, wherein the main treatment process includes a first treatment process that alternately performs the first lighting operation and an unlit operation, and a second treatment process that alternately performs the second lighting operation and an unlit operation or that continuously performs the second lighting operation.

14. The method of inactivation treatment for bacteria and/or viruses according to claim 9 or 10, wherein the main treatment process includes a third treatment process that alternately performs the first lighting operation and the second lighting operation, and a fourth treatment process that alternately performs the second lighting operation and the first lighting operation, and that has a higher frequency of the second lighting operation per unit time compared to the third treatment process.

15. The method of inactivation treatment for bacteria and/or viruses according to claim 9, further comprising a secondary treatment process that performs ultraviolet light irradiation different from that of the main treatment process upon a detection of satisfying a predetermined condition.
